# EUROPEAN PATENT SPECIFICATION

(11) **EP 2 441 488 B1**
(45) Date of publication and mention of the grant of the patent: **31.08.2016**
(21) Application number: 11184570.7
(22) Date of filing: 11.10.2011
(51) Int. Cl.: A61M 29/00, A61M 25/10, A61M 31/00, A61J 15/00, A61B 17/34, A61M 25/06, A61B 17/00, A61M 25/00

(54) **BALLOON CATHETER SYSTEM AND METHOD OF ASSEMBLY**
BALLONKATHETERSYSTEM UND VERFAHREN ZUR KONSTRUKTION
SYSTÈME DE CATHÉTER À BALLONNET ET PROCÉDÉ D'ASSEMBLAGE

(30) Priority: 15.10.2010 US 905522
(43) Date of publication of application: 18.04.2012
(60) Divisional application: 15202820.5
(73) Proprietor: Nath, Iyunni Venkata Sesha Sayi, Seminole, FL 33777 (US)
(72) Inventor: Nath, Iyunni Venkata Sesha Sayi, Seminole, FL 33777 (US)
(74) Representative: Maiwald Patentanwalts GmbH

(56) References cited:
- EP-A1- 0 266 469
- EP-A2- 1 293 228
- WO-A1-2006/087512
- WO-A1-2010/108678
- WO-A2-2005/112801
- US-A- 4 698 056

## Description

### Field of the Invention

The field relates to a balloon catheter system for insertion into a patient using an endoscopic percutaneous insertion method or minimally invasive procedures.

### Background

The Applicant claims the benefit of and priority to U.S. Pat. Appl. Ser. No. 61/031,442 filed February 26, 2008 and U.S. Pat. Appl. Ser. No. 61/113,697 filed November 12, 2008 and U.S. Pat. Appl. Ser. No. 12/393,717 filed February 26, 2009. In the following these applications will be referenced as the "Nath Application". The state of the art does not disclose a balloon catheter system according to claim 1 with a catheter for use in a percutaneous, minimally invasive procedure that utilizes a detachable dilator. Also, the existing devices are not capable of being used effectively for procedures as varied as gastric feeding, gastric decompression, jejunal feeding, cystostomy, nephrostomy, weight loss and colostomy.

WO 2006/087512 A1 relates to a medico-surgical apparatus that has a tube with an inflatable sealing cuff, a tubular stem in the tube and a dilator at an end of the tube, which is interconnected with the tubular stem.

### Summary

It is the object of the invention to achieve the above mentioned needs. This object is met by the subject-matter of the independent claim. Further exemplary embodiments are evident from the dependent claims. In the following description, examples are described which may not directly relate to the invention, but which may be useful in connection with the invention.

The examples of the invention comprise a balloon catheter for use in a wide variety of procedures including, without limitation, one or more of the following procedures: gastric feeding, gastric decompression, jejunal feeding, cystostomy, bladder decompression, colonoscopy, nephrostomy and weight loss. In one example, a single balloon device may be used for all of the following indications for use: gastric feeding, gastric decompression, cystostomy, bladder decompression, nephrostomy and colostomy. In another example, a double balloon device may be used for one or more of these and for weight loss. In yet another example, a device including a plurality of balloons may be used for one or more on the following: gastric feeding, gastric decompression, jejunal feeding and weight loss.

For example, a catheter includes a balloon inflatable using a fluid, such as water or saline, which is capable of acting to retain the catheter in a cavity or lumen, such as the stomach, bladder or colon, for example. When inflated, the balloon is pulled against the wall of the lumen and seals the lumen. The catheter may be inserted under observation using minimally invasive techniques, such as an endoscope. The procedure is modified from the process disclosed in the Nath Application. Instead, a detachable dilator is arranged on the end of the catheter such that an introducer needle, shaft or rod extends through the detachable dilator. The dilator may be detached and retrieved using a snare, which may be used to remove the dilator through an endoscopic passage or other minimally invasive method. The detachable dilator of the invention is made of a material that is capable of being bioabsorbed or may be capable of being detached from the catheter and passing without adverse consequences. For example, certain starch-based materials and gels are capable of being bioabsorbed and/or passing without adverse consequences. Also, it is believed, without being limiting in any way, that a silicone rubber dilator would be capable of passing through the digestive tract without adverse consequences. Other materials for the dilator tip may be a PEEK or a polyethylene, such as high density polyethylene. PEEK is a polyether ether ketone, which may be colorless, and is a known biocompatible engineering thermoplastic material.

The system provides advantages over any known method for inserting a comparatively large catheter, which may be any type of tube, into a cavity or lumen in a patient, which may be a human patient or a non-human patient, such as an animal. Insertion of the catheter is greatly simplified using the detachable dilator. A guide wire, preferably a soft tip guide wire, is inserted in a location where catheterization is desired, using a trochar or other device as is known in the art. The end of the guide wire opposite of the end inserted into the patient is inserted into an annular shaft that extends through the catheter and at least a portion of the dilator. The annular shaft, dilator and catheter tube may be pre-assembled. The assembly may be guided along the guidewire and into the patient, with or without lubrication of the catheter.

For example, the annular shaft may be an introducer needle, which may be made of any stiff, biocompatible material, such as a metal. In one example, the metal is stainless steel. The introducer needle is used only during insertion and is removed after the catheter is properly positioned within the patient.

The dilator follows the guidewire into the patient, widening the entrance by its transition from about the same diameter as the guide wire or the annular shaft to a diameter about the same diameter or larger than the outer diameter of the catheter. For example, the shape of the nose of the dilator may be a cone, truncated cone or paraboloid. A substantially conical outer surface of a dilator nose may be used, for example. Alternatively, the outer surface of the dilator nose may have a substantially parabolic shape. One advantage of the use of a detachable dilator is that the dilator may have an integrated sheath protecting a balloon of a balloon catheter during insertion of the balloon catheter. Another advantage of a detachable dilator is that the dilator is not withdrawn through the catheter, itself. Therefore, the dilator may have an outer diameter larger than the inner diameter of the tubular device being inserted into a patient. However, unlike external dilators, the catheter need not be inserted through the dilator. Instead, the nose of the dilator may be sized to be the same external diameter as the external diameter of the catheter, because the dilator is dislodged from the catheter after the catheter is properly positioned in the patient. Surprisingly, the detachable dilator reduces the time to perform an insertion procedure while avoiding, or at least reducing, leakage from the cavity around the point of insertion during an insertion procedure.

Surprisingly, a colostomy procedure may use a balloon catheter with a detachable dilator to quickly and efficiently decompress, cleanse and treat a patient that would otherwise be faced with a colectomy. The colostomy uses the minimally invasive surgical techniques similar to a gastrostomy or cystostomy to introduce a balloon catheter in the colon. The balloon catheter may be used for decompression and drainage by connecting the catheter with a colostomy bag. In addition, access to the colon is provided using the catheter for treating an infection or the like. It is surprising and unexpected that essentially the same procedure may be used in not only colostomy but also for nephrostomy, cystostomy, gastrostomy and weight loss. The additional steps required for jejunostomy are the same as required for any jejunostomy procedure. The insertion procedure has fewer complications than known insertion procedures, requires fewer devices to be stocked and improves proficiency by providing a device capable of being used in a plurality of "ostomy" procedures.

A double balloon catheter may be inserted into a patient's stomach in a gastrostomy procedure and may be used for weight loss. By inflating the two balloons, hunger is alleviated, helping the patient to lose weight and to gradually reduce the size of the stomach, while avoiding any blockage of nutrition and hydration. In one example, supplemental nutrition and/or hydration may be provided by gastric feeding, as prescribed by a physician. The dumbbell-shaped double balloon provides a channel for passage of nutrition and hydration through the stomach, while occupying space in the stomach, alleviating discomfort from an empty feeling in the stomach.

In the embodiment of the invention, a balloon catheter system for insertion into a patient comprises a balloon catheter, an annular shaft and a detachable dilator. The balloon catheter may be comprised of a tube having a first end, a second end opposite of the first end, a central bore fluidically coupling an inlet at the first end of the tube with an outlet at the second end of the tube and a lumen fluidically coupling a connector disposed adjacent the first end of the tube with a balloon disposed adjacent to the second end of the tube, the balloon being formed by a tubular film sealed at opposite ends of the tubular film to an external surface of the tube. The lumen has a lumen outlet such that fluid injected into the connector and through the lumen exits through the external surface of the tube at the outlet and into an expandable cavity between the balloon, which is made of a flexible, resilient material, and the external surface of the tube. The balloon is capable of being expanded by injecting fluid into the expandable cavity through the lumen.

The detachable dilator may be coupled to the outlet of the tube, when assembled, such that the dilator is held in place during insertion of the tube. After the tube is inserted, the dilator is detached from the tube. The dilator may comprise a sheath portion and a nose, the nose having an external surface that transitions from a first external diameter at a nose tip to a second external diameter at a portion of the nose adjacent to the outlet of tube, when the dilator is coupled to the outlet of the tube. An internal surface of the nose provides the channel through which the annular shaft may be inserted. The sheath portion of the dilator may be formed of a flexible, resilient material, and may be formed in a shape such that it closes in on itself, when the dilator is detached from the tube. The entire dilator is made of a digestible material and/or bioabsorbable material.

The annular shaft may extend entirely through the nose or may extend through only a portion of the nose of the dilator. For example, the annular shaft may butt up against a portion of the nose of the dilator at a constriction formed within the inner surface of the nose, and the dilator may be detachable from the tube using only the annular shaft, such as by pushing the annular shaft to force the dilator off of the end of the catheter tube. In one example, the annular shaft is attached to a control mechanism, allowing the annular shaft, which extends only partially through the nose, to be used to push the detachable dilator off of the catheter tube, after insertion of the catheter tube into the patient, when the control mechanism is activated. The annular shaft extends through the central bore of the tube and is capable of being removed from the central bore of the tube, after the tube is inserted into the patient, by pulling on an extraction portion of the annular shaft. The extraction portion may extend from the inlet of the tube or may be attached to an extractor that extends from the inlet of the tube. The annular shaft may be made of a stainless steel, for example. In one example, the annular shaft has a pointed tip extending from the nose of the dilator, when the dilator is coupled to the outlet of the tube.

The balloon catheter system may comprise a separate detachment mechanism in addition to the catheter tube, annular shaft and dilator. For example, the detachment mechanism may comprise a tubular annulus having an inner bore diameter fitting over the outer diameter of the annular shaft and a distal end capable of butting up against the nose of the dilator. By pushing the tubular annulus the detachment mechanism is capable of detaching the dilator from the tube of the catheter, for example. The detachment mechanism may include a control mechanism at a proximal end of the tubular annulus of the detachment mechanism and may include a locking mechanism, which positively locks the tubular annulus until the locking mechanism is unlocked, preventing the distal end of the tubular annulus from pushing the dilator. For example, the locking mechanism may prevent the tubular annulus from being rotated about the longitudinal axis of the tubular annulus. If rotated, about its axis, a guide may be aligned to engage channel allowing the tubular annulus to engage the nose of the dilator, pushing the dilator from the tube of the catheter.

A method of assembling the balloon catheter system may comprise inserting the annular shaft through the central bore of the tube and through at least a portion of the channel of the nose, and coupling the dilator to the outlet of the tube such that the sheath portion of the dilator extends over the outlet of the tube and over at least a portion of the balloon. If the sheath portion of the dilator is formed of a flexible, resilient material, which is closes in on itself, then prior to coupling the dilator to the outlet of the tube, the sheath portion may be unfolded and spread to allow insertion of the outlet portion of the tube into the sheath portion of the dilator.

### Brief Description of the Drawings

Figures 1A - 1C illustrate a detachable dilator as seen (A) externally and (B) in a cross sectional view, while the sheath portion is spread for insertion of the catheter tube, and (C) in a cross sectional view with the sheath portion folded in on itself.
Figure 2 illustrates an example of the use of a detachable dilator, shown in cross section, fitted with a needle introducer and a balloon catheter.
Figure 3 illustrates another example of the use of a detachable dilator, shown in cross section, fitted with a needle introducer, a balloon catheter, in partial cross section, and an example of a detacher.
Figure 4 illustrates a perspective view of an example of a detacher head.
Figure 5 illustrates a perspective view of an example of a detacher handle.
Figure 6 illustrates a partially exploded view of a detacher head, a detacher handle and a needle introducer.

### Detailed Description

The examples described and the drawings rendered are illustrative and are not to be read as limiting the scope of the invention as it is defined by the appended claims.

The method of insertion of a balloon catheter using a detachable dilator is similar to the procedure used in the Nath Application. However, instead of withdrawing the needle with a flexible sheath through the large channel in the feeding tube of the Nath Application, only the needle introducer is withdrawn through the catheter. The dilator is detached from the catheter after insertion of the catheter. In one example, the dilator is made of a biocompatible and/or digestible polymer. For example, a polytetrafluoroethylene may be used for the dilator and provides exceptional biocompatibility together with advantageous tribology. However, other polymer materials may be used, such as polyvinylchloride, polyethylene, polypropylene, polyethylene terephthalate, other polyesters, polymethylmethacrylate, polycarbonates, polyamides, polyacetals, polysulfones and co-polymers, such as tetrafluoroethylene and hexafluoropropylene and polyurethane block co-polymers.

In one example, the dilator is withdrawn using minimally invasive techniques. For example, a snare, such as one of the net-like snares capable of being used with an endoscope, is used to capture and remove the dilator. For example, a Roth Net^{®} foreign body standard snare may be used.¹
¹ROTH NET^{®} is a trademark of US Endoscopy.

In the embodiment of the invention, the dilator is not withdrawn but is made of a bioabsorbable or digestible material or a material that passes harmlessly through the digestive tract. For example, a dilator may be made of a polylactide, which exhibits high tensile strength and rigidity, but hydrolyzing breaks the polylactide down to lactic acid, which digestible or passes harmlessly through the digestive tract. Other bioabsorbable materials include poly(trimethyl carbonate), poly(carbonate) and starch-based polymers and gels, which may provide sufficient rigidity to serve as a dilator.

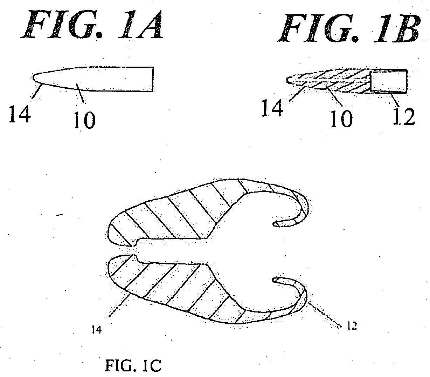

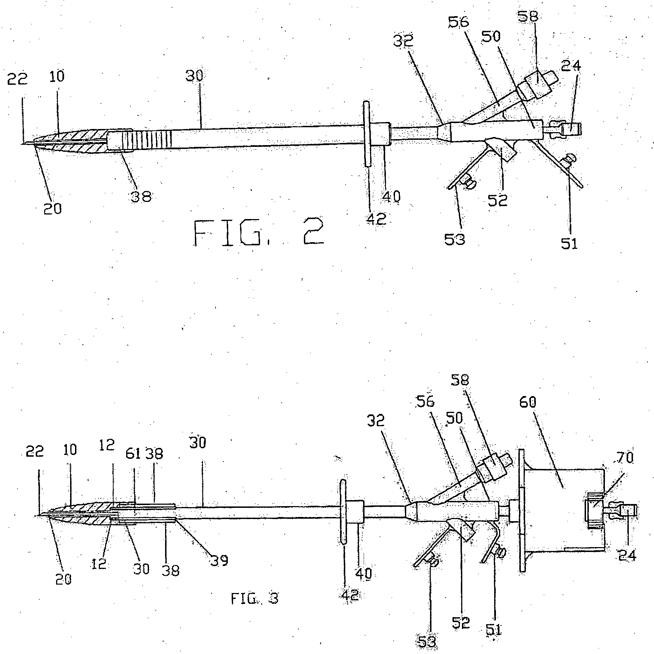

**Figure 1A** illustrates a dilator **10,** having an asymmetric nose **14** and an integrally formed protective sheath **12,** as illustrated in the cross sectional view of **Figure 1B**. In **Figure 1C****,** a resilient, flexible material, such as PEEK or silicone rubber, is used to form the sheath portion of the dilator, and the sheath portion is capable of returning to a shape folded in on itself. This may make it possible for the dilator to pass through the digestive tract with no harm to the patient, for example, and reduces the length of the dilator, if it is to be captured by a net, for example. **Figure 2** illustrates use of the detachable dilator **10** with a needle introducer **20** and a tri-funnel balloon catheter **30.** The needle introducer is inserted through a channel defined by the inner diameter of the annular dilator **10**. In one example, the needle introducer **20** and dilator **10** are sized, such that the needle introducer may be withdrawn from the dilator **10** and the catheter **30** by pulling the protector **24** while pushing on the catheter **30** to keep the catheter **30** stationary. In this way, both the needle introducer **20** and a guide wire inserted through the needle introducer may be withdrawn from the catheter **30.** The dilator **10** is then free to be detached from the catheter **30.** In one example, an integrally formed **sheath 12** is thin, such as less than 1 millimeter in thickness, and non-rigid, and the dilator **10** dislodges from the catheter **30** as soon as the needle introducer **10** is removed. Alternatively, the dilator **10** may be dislodged from the catheter **30** by expanding the balloon **38,** which is attached at the end of the catheter **30** by adhesive bonds **39** on each end of the balloon, for example. A balloon lumen **56** may be coextruded such that fluid inserted by syringe at the luer lock **58** inflates the balloon with a liquid, such as water or saline. A plurality of balloons may be provided by having additional coextruded balloon lumen, for example. The sheath **12** may protect each of the plurality of balloons, a single balloon or a portion of a single balloon. **Figure 2** illustrates a sheath protecting a leading edge of a balloon **38,** for example.

An external bolster **40** provides a flange **42** that may be slid downward, once the balloon **38** is inflated and the catheter **30** is in place. In the example of **Figures 2** and **3****,** a tri-funnel connector **50** is attached to the tube of the catheter by a transition region **32.** The catheter has a main feeding channel, which may be closed by a flexibly attached plug **51** and a secondary drainage port **52,** which may be connected to a drainage bag. The main channel may be flushed with sterile water or saline to keep the catheter free of obstructions and clots. A second plug **53** may be used to close the drainage port **52,** when it is not being used. A luer lock **58** seals the balloon inflation lumen **56,** which may be used to fill or drain the balloon **38** using a syringe.

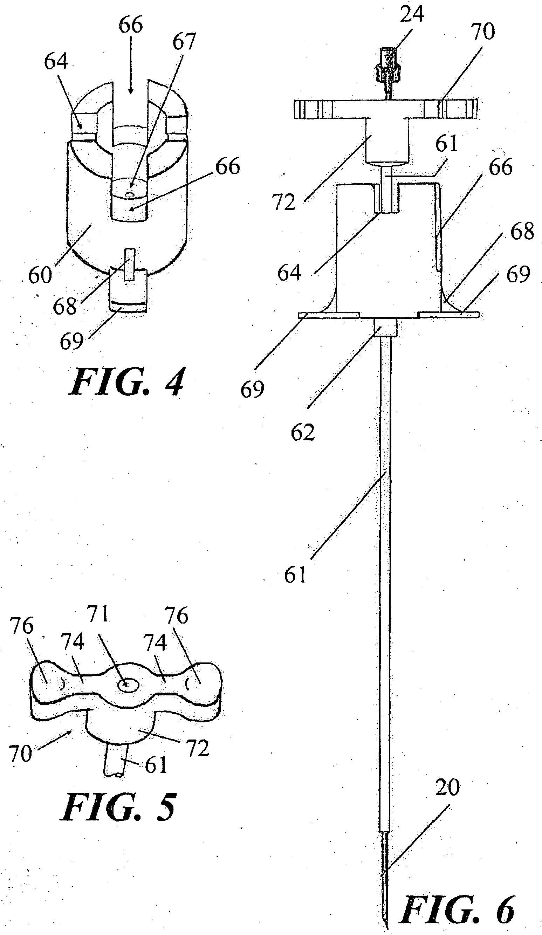

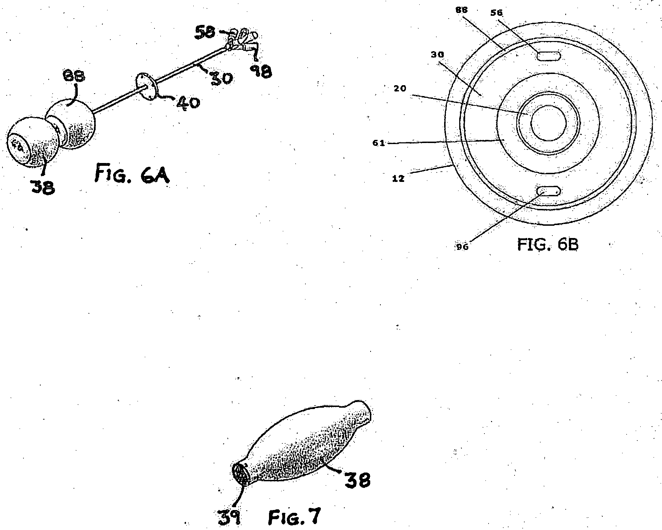

**Figure 3** illustrates the use of a detachment mechanism **60, 70,** which includes a head **60,** a handle **70** and a hollow push rod **61,** which has a channel through which the needle introducer **20** extends, when preassembled prior to insertion, for example. As illustrated in **Figure 4****,** the head **60** has finger holds **69** on one end and slots or channels **64, 66** on the opposite end. The slots **64, 66** are dimensioned to accommodate the narrow portions **74** of the handle **70.** A central bore **67** of the head **60** is sized to accommodate the diameter of the push rod **61.** The push rod **61** is coupled to the handle **70** and one end of the push rod **61** extends from one side of the handle **70.** The opposite end of the push rod **61** is capable of contacting the dilator **10** to force detachment of the dilator **10** from the catheter **30,** when the handle **70** is backed-off, and rotated 90 degrees in the head **60,** as illustrated in **Figure 6****,** aligning the narrow portion **74** of the handle **70** with the elongated channels **66.** The user's fingers may compress the finger holds **76** of the handle **70** toward the finger holds **69** of the head **60,** which are reinforced by arcuate supports **68**. The head **60** has a funnel shape surrounding the central bore **67,** which provides a centering function when the push rod **61** is inserted into the central bore **67.** A tapered retention plug **62** extends from the tubeward side of the head **60** and is capable of mating with the main channel of the tri-funnel connector **50** as illustrated in **Figure 3****.** A slightly tapered plunger barrel **72** extends into the head **60** and acts to guide the handle **70** into the head **60** during compression. The handle **70** includes a central bore **71** that is capable of accommodating the needle introducer **20,** as illustrated in **Figure 6****.** The protector **24** on the outward end of the needle introducer **20** may be held with one hand, while the other hand is used to plunge the handle into the head, keeping the needle introducer **20** in place while the dilator **10** is detached from the catheter **30.** Alternatively, the needle introducer may be withdrawn prior to detaching the dilator **10** using the detachment mechanism **60, 70.**

In Figure 6A-6B, a double balloon (dumbbell-shaped) catheter is illustrated which may not directly be related to the invention, but which may be useful in connection with the invention. The addition of a second balloon 88, in addition to the balloon nearest the outlet of the catheter 38, is provided to use in patients where a single balloon is insufficient. One or both of the balloons may have a larger volume than a single balloon device. For example, if a single balloon device has a balloon is filled with 20 cubic centimeters of liquid, and then one or both of the double balloons may be filled with 40 cubic centimeters of liquid. Alternatively, each balloon may be filled with the same volume of liquid, for example 20 cubic centimeters. In one example, each of the balloons in the double balloon device are capable of being filled to a maximum volume of 120 cubic centimeters, and use of the catheter is indicated for obese patients that have failed to control weight through diet and exercise. Each balloon has its own, independent luer lock 58,98 for expansion and contraction of the balloon volumes in Figures 6A - 6B. The gap between the two balloons 38, 88 provides a path for food and liquid to pass from the esophagus, through the stomach and to the intestines. A feeding port and drainage port are provided as illustrated in the single balloon catheter of Figures 2-3.

A typical balloon 38 is illustrated in Figure 7, and the adhesive layer 39 is represented by the cross-hatched region on the end of the balloon.

## Claims

1. A balloon catheter system for insertion into a patient comprising:
a balloon catheter (30) comprised of a tube having a first end, a second end opposite of the first end, a central bore fluidically coupling an inlet at the first end of the tube with an outlet at the second end of the tube and a lumen fluidically coupling a connector disposed adjacent the first end of the tube with a balloon (38) disposed adjacent to the second end of the tube, the balloon (38) being formed by a tubular film sealed at opposite ends of the tubular film to an external surface of the tube, the lumen having a lumen outlet such that fluid injected into the connector and through the lumen exits through the external surface of the tube at the outlet and into an expandable cavity between the balloon (38), which is made of a flexible, resilient material, and the external surface of the tube, whereby the balloon (38) is capable of being expanded by injecting fluid into the expandable cavity through the lumen;
a detachable dilator (10) adapted for being coupled to the outlet of the tube, such that the dilator (10) is held in place during insertion of the tube, and adapted for being detached from the tube after the tube is inserted, wherein the dilator (10) comprises a sheath portion (12) and a nose (14), the nose (14) having an external surface that transitions from a first external diameter at a nose tip to a second external diameter at a portion of the nose adjacent to the outlet of tube, when the dilator (10) is coupled to the outlet of the tube, and an internal surface providing a channel through the nose (14), the sheath portion (12) extending from the nose (14) and over the outlet of the tube and over at least a portion of the balloon (38), the dilator being made of a digestible material and/or a bioabsorbable material; and
an annular shaft (20, 61) adapted for being inserted through the central bore of the tube and through at least a portion of the channel of the nose (14), when the dilator (10) is coupled to the outlet of the tube, and the annular shaft (20) is capable of being removed from the central bore of the tube, after the tube is inserted into the patient, by pulling on an extraction portion (24) of the annular shaft (20, 61) extending from the inlet of the tube.

2. The balloon catheter system of claim 1, wherein the annular shaft (20) is of a stainless steel.

3. The balloon catheter system of claim 2, wherein the annular shaft (20) has a pointed tip (22) extending from the nose (14) of the dilator (10), when the dilator (10) is coupled to the outlet of the tube.

4. The balloon catheter system of claim 1, wherein the dilator (10) is detachable from the tube by using only the annular shaft (20).

5. The balloon catheter system of claim 1, further comprising a detachment mechanism (60, 70), the detachment mechanism comprising a tubular annulus (61) having an inner bore diameter fitting over the annular shaft (20) and a distal end capable of contacting the dilator (10), whereby the detachment mechanism (60, 70) is capable of detaching the dilator (10) from the tube.

6. The balloon catheter system of claim 5, wherein the detachment mechanism (60, 70) includes a control mechanism at a proximal end of the tubular annulus (61) of the detachment mechanism.

7. The balloon catheter system of claim 6, wherein the control mechanism includes a locking mechanism.

8. The balloon catheter system of claim 7, wherein the control mechanism prevents the distal end from pushing the dilator (10), whereby the dilator (10) is detached from the tube, until the tubular annulus (61) is rotated about a longitudinal axis of the tubular annulus (61).

9. The balloon catheter system of claim 1, wherein the sheath portion (12) of the dilator (10) is formed of a flexible, resilient material, and the sheath portion (12) closes in on itself, when the dilator (10) is detached from the tube.

10. A method of assembling the balloon catheter system of claim 1, comprising:
inserting the annular shaft (20, 61) through the central bore of the tube and through at least a portion of the channel of the nose (14) of the dilator (10); and
coupling the dilator (10) to the outlet of the tube such that the sheath portion (12) of the dilator extends over the outlet of the tube and over at least a portion of the balloon (38).

11. The method of claim 10, wherein the sheath portion (12) of the dilator (10) is formed of a flexible, resilient material, and the sheath portion (12) closes in on itself, prior to coupling the dilator (10) to the outlet of the tube; and the step of coupling includes unfolding and spreading the sheath portion (12) and inserting the outlet of the tube into the sheath portion (12) of the dilator (10).

## Patentansprüche

1. Ballonkathetersystem zum Einführen in einen Patienten, umfassend:
einen Ballonkatheter (30) aufweisend eine Röhre mit einem ersten Ende, einem zweiten Ende gegenüber dem ersten Ende, einer zentralen Bohrung, welche einen Einlass an dem ersten Ende der Röhre mit einem Auslass an dem zweiten Ende der Röhre strömungstechnisch koppelt und ein Lumen, welches einen angrenzend zu dem ersten Ende der Röhre angeordneten Verbinder mit einem angrenzend zu dem zweiten Ende der Röhre angeordneten Ballon (38) strömungstechnisch koppelt, wobei der Ballon (38) durch einen röhrenförmigen Film gebildet wird, welcher an gegenüberliegenden Enden des röhrenförmigen Films zu einer äußeren Oberfläche der Röhre abgedichtet ist, wobei das Lumen einen Lumenauslass hat, so dass in den Verbinder eingespritztes und durch das Lumen hindurchtretendes Fluid durch die äußere Oberfläche der Röhre am Auslass und in einen expandeierbaren Hohlraum zwischen dem Ballon (38), welcher aus einem flexiblen, elastischen Material gefertigt ist, und der äußeren Oberfläche der Röhre entweicht, wobei der Ballon (38) imstande ist, durch Einspritzen von Fluid in den expandierbaren Hohlraum durch den Lumen zu expandieren;
einen lösbaren Dilatator (10), welcher dazu ausgeführt ist, mit dem Auslass der Röhre gekoppelt zu werden, so dass der Dilatator (10) während des Einsetzens der Röhre in Position gehalten wird, und der dazu ausgeführt ist, von der Röhre gelöst zu werden, nachdem die Röhre eingeführt wird, wobei der Dilatator (10) einen Mantelabschnitt (12) und eine Nase (14) mit einer äußeren Oberfläche aufweist, welche von einem ersten Außendurchmesser an der Nasenspitze zu einem zweiten Außendurchmesser an einem Abschnitt der Nase angrenzend zum Auslass der Röhre übergeht, wenn der Dilatator (10) mit dem Auslass der Röhre gekoppelt ist, und mit einer inneren Oberfläche, welche einen Kanal durch die Nase (14) bereitstellt, wobei sich der Mantelabschnitt (12) von der Nase (14) über den Auslass der Röhre und über mindestens einen Abschnitt des Ballons (38) erstreckt, wobei der Dilator aus einem verdaulichen und/oder einem biologisch abbaubaren Material gefertigt ist; und
eine ringförmige Welle (20, 61), die dazu ausgeführt ist, um durch die zentrale Bohrung der Röhre und durch zumindest einen Abschnitt des Kanals der Nase (14) eingeführt zu werden, wenn der Dilatator (10) mit dem Auslass der Röhre gekoppelt ist, wobei die ringförmige Welle (20) von der zentralen Bohrung der Röhre entfernbar ist, nachdem die Röhre in den Patienten eingeführt wird, indem an einem sich vom Enlass der Röhre erstreckenden Extraktionsabschnitt (24) der ringförmigen Welle (20, 61) gezogen wird.

2. Ballonkathetersystem nach Anspruch 1, wobei die ringförmige Welle (20) aus einem rostfreiem Stahl ist.

3. Ballonkathetersystem nach Anspruch 2, wobei die ringförmige Welle (20) eine sich von der Nase (14) des Dilatators (10) erstreckende, spitz zulaufende Spitze (22) hat, wenn der Dilatator (10) mit dem Auslass der Röhre gekoppelt ist.

4. Ballonkathetersystem nach Anspruch 1, wobei der Dilator (10) von der Röhre durch Verwenden lediglich der ringförmigen Welle (20) lösbar ist.

5. Ballonkathetersystem nach Anspruch 1, ferner aufweisend einen Lösemechanismus (60, 70), welcher einen röhrenförmigen Ringraum (61) mit einem Innenbohrungsdurchmesser aufweist, der über die ringförmige Welle (20) passt und ein distales Ende zum Kontaktieren des Dilators (10) aufweist, wobei der Lösemechanismus (60, 70) imstande ist, den Dilator (10) von der Röhre zu lösen.

6. Ballonkathetersystem nach Anspruch 5, wobei der Lösemechanismus (60, 70) einen Steuermechanismus an einem proximalen Ende des röhrenförmigen Ringraums (61) des Lösemechanismus aufweist.

7. Ballonkathetersystem nach Anspruch 6, wobei der Steuermechanismus einen Verriegelungsmechanismus aufweist.

8. Ballonkathetersystem nach Anspruch 7, wobei der Steuermechanismus verhindert, dass das distale Ende den Dilator (10) anstößt, wobei der Dilatator (10) von der Röhre gelöst ist, bis sich der röhrenförmige Ringraum (61) um eine Längsachse des röhrenförmigen Ringraums (61) dreht.

9. Ballonkathetersystem nach Anspruch 1, wobei der Mantelabschnitt (12) des Dilatators (10) aus einem flexiblen, elastischen Material gebildet ist, und der Mantelabschnitt (12) sich selbst zuzieht, wenn der Dilator (10) von der Röhre gelöst wird.

10. Verfahren zum Zusammenbauen des Ballonkathetersystems nach Anspruch 1, aufweisend:
Einführen der ringförmigen Welle (20, 61) durch die zentrale Bohrung der Röhre und durch zumindest einen Abschnitt des Kanals der Nase (14) des Dilators (10); und Koppeln des Dilatators (10) an den Auslass der Röhre, so dass sich der Mantelabschnitt (12) des Dilatators über den Auslass der Röhre und über zumindest einen Abschnitt des Ballons (38) erstreckt.

11. Verfahren nach Anspruch 10, wobei der Mantelabschnitt (12) des Dilatators (10) aus einem flexiblen, elastischen Material gebildet ist, und der Mantelabschnitt (12) sich selbst zuzieht, bevor der Dilatator (10) mit dem Auslass der Röhre gekoppelt wird; und wobei der Schritt des Koppelns ein Entfalten und Ausbreiten des Mantelabschnitts (12) und ein Einführen des Auslasses der Röhre in den Mantelabschnitt (12) des Dilatators (10) umfasst.

## Revendications

1. Système de cathéter à ballonnet destiné à une insertion dans un patient, comportant :
un cathéter à ballonnet (30) constitué d'un tube ayant une première extrémité, une seconde extrémité opposée à la première extrémité, un trou central couplant fluidiquement une entrée sur la première extrémité du tube avec une sortie sur la seconde extrémité du tube et une lumière couplant fluidiquement un raccord disposé adjacent à la première extrémité du tube avec un ballonnet (38) disposé adjacent à la seconde extrémité du tube, le ballonnet (38) étant formé par un film tubulaire fermé sur des extrémités opposées du film tubulaire à une surface externe du tube, la lumière ayant une sortie de lumière de telle sorte que du fluide injecté dans le raccord et à travers la lumière sort par la surface externe du tube au niveau de la sortie et dans une cavité expansible entre le ballonnet (38), qui est constitué d'un matériau flexible et élastique, et la surface externe du tube, de sorte que le ballonnet (38) peut s'expanser en injectant du fluide dans la cavité expansible à travers la lumière,
un dilatateur détachable (10) adapté pour être couplé à la sortie du tube, de telle sorte que le dilatateur (10) est maintenu en place pendant l'insertion du tube, et adapté pour être détaché du tube après que le tube ait été inséré, dans lequel le dilatateur (10) comporte une partie de fourreau (12) et un nez (14), le nez (14) ayant une surface externe qui passe d'un premier diamètre externe sur une pointe de nez à un second diamètre externe sur une partie du nez adjacente à la sortie du tube, lorsque le dilatateur (10) est couplé à la sortie du tube, et une surface interne fournissant un canal à travers le nez (14), la partie de fourreau (12) s'étendant à partir du nez (14) et au-dessus de la sortie du tube et au-dessus d'au moins une partie du ballonnet (38), le dilatateur étant constitué d'un matériau digestible et/ou d'un matériau bioabsorbable, et
une tige annulaire (61) adaptée pour être insérée à travers le trou central du tube et à travers au moins une partie du canal du nez (14), lorsque le dilatateur (10) est couplé à la sortie du tube, et la tige annulaire (20) peut être retirée du trou central du tube, après que le tube ait été inséré dans le patient, en tirant sur une partie d'extraction (24) de la tige annulaire (20, 61) s'étendant à partir de l'entrée du tube.

2. Système de cathéter à ballonnet selon la revendication 1, dans lequel la tige annulaire (20) est en acier inoxydable.

3. Système de cathéter à ballonnet selon la revendication 2, dans lequel la tige annulaire (20) a un bout pointu (22) s'étendant à partir du nez (14) du dilatateur (10), lorsque le dilatateur est couplé à la sortie du tube.

4. Système de cathéter à ballonnet selon la revendication 1, dans lequel le dilatateur (10) peut être détaché du tube en utilisant uniquement la tige annulaire (20).

5. Système de cathéter à ballonnet selon la revendication 1, comportant en outre un mécanisme de détachement (60, 70), le mécanisme de détachement comportant un anneau tubulaire (61) ayant un diamètre de trou s'adaptant sur la tige annulaire (20) et une extrémité distale pouvant venir en contact avec le dilatateur (10), de sorte que le mécanisme de détachement (60, 70) peut détacher le dilatateur (10) du tube.

6. Système de cathéter à ballonnet selon la revendication 5, dans lequel le mécanisme de détachement (60, 70) comprend un mécanisme de commande sur une extrémité proximale de l'anneau tubulaire (61) du mécanisme de détachement.

7. Système de cathéter à ballonnet selon la revendication 6, dans lequel le mécanisme de commande comprend un mécanisme de verrouillage.

8. Système de cathéter à ballonnet selon la revendication 7, dans lequel le mécanisme de commande empêche l'extrémité distale de pousser le dilatateur (10), de sorte que le dilatateur (10) est détaché du tube, jusqu'à ce que l'espace annulaire tubulaire (61) tourne autour d'un axe longitudinal de l'espace annulaire tubulaire (61).

9. Système de cathéter à ballonnet selon la revendication 1, dans lequel la partie de fourreau (12) du dilatateur (10) est formée d'un matériau flexible et élastique, et la partie de fourreau (12) se ferme sur elle-même, lorsque le dilatateur (10) est détaché du tube.

10. Procédé d'assemblage du système de cathéter à ballonnet selon la revendication 1, comportant les étapes consistant à :
insérer la tige annulaire (20, 61) à travers le trou central du tube et à travers au moins une partie du canal du nez (14) du dilatateur (10), et
coupler le dilatateur (10) à la sortie du tube de telle sorte que la partie de fourreau (12) du dilatateur s'étend au-dessus de la sortie du tube et au-dessus d'au moins une partie du ballonnet (38).

11. Procédé selon la revendication 10, dans lequel la partie de fourreau (12) du dilatateur (10) est formée d'un matériau flexible et élastique, et la partie de fourreau (12) se ferme sur elle-même, avant de coupler le dilatateur (10) à la sortie du tube, et l'étape de couplage comprend le dépliage et l'écartement de la partie de fourreau (12) et l'insertion de la sortie du tube dans la partie de fourreau (12) du dilatateur (10).
